# EUROPEAN PATENT SPECIFICATION

(11) **EP 4 172 626 B1**
(45) Date of publication and mention of the grant of the patent: **28.08.2024**
(21) Application number: 21733361.6
(22) Date of filing: 29.06.2021
(51) Int. Cl.: G01N 33/564, G01N 33/68

(54) **PROGNOSIS OF CHRONIC INFLAMMATORY DEMYELINATING POLY-NEUROPATHY**
PROGNOSE VON CHRONISCHER ENTZÜNDLICHER DEMYELINISIERENDER POLYNEUROPATHIE
PRONOSTIC DE POLYNEUROPATHIE DÉMYÉLINISANTE INFLAMMATOIRE CHRONIQUE

(30) Priority: 29.06.2020 GB 202009864; 08.03.2021 GB 202103184
(43) Date of publication of application: 03.05.2023
(73) Proprietor: Katholieke Universiteit Leuven, KU Leuven R&D, 3000 Leuven (BE)
(72) Inventor: POESEN, Koen, 3020 Herent (BE)
(74) Representative: Arnold & Siedsma
(86) International application number: PCT/EP2021/067769
(87) International publication number: WO 2022/002889

(56) References cited:
- LIEVERLOO GWEN G. A. ET AL: "Serum neurofilament light chain in chronic inflammatory demyelinating polyneuropathy", JOURNAL OF THE PERIPHERAL NERVOUS SYSTEM, vol. 24, no. 2, 29 April 2019 (2019-04-29), US, pages 187 - 194, XP055848474, ISSN: 1085-9489, Retrieved from the Internet <URL:https://onlinelibrary.wiley.com/doi/full-xml/10.1111/jns.12319> DOI: 10.1111/jns.12319
- MARIOTTO SARA ET AL: "Serum and cerebrospinal neurofilament light chain levels in patients with acquired peripheral neuropathies", JOURNAL OF THE PERIPHERAL NERVOUS SYSTEM, vol. 23, no. 3, 24 July 2018 (2018-07-24), US, pages 174 - 177, XP055848554, ISSN: 1085-9489, Retrieved from the Internet <URL:https://onlinelibrary.wiley.com/doi/full-xml/10.1111/jns.12279> DOI: 10.1111/jns.12279
- KHALIL MICHAEL ET AL: "Neurofilaments as biomarkers in neurological disorders", NATURE REVIEWS. NEUROLOGY, NATURE, US, vol. 14, no. 10, 31 August 2018 (2018-08-31), pages 577 - 589, XP036601948, ISSN: 1759-4758, [retrieved on 20180831], DOI: 10.1038/S41582-018-0058-Z
- HÄRTIG FLORIAN ET AL: "Nerve Ultrasound Predicts Treatment Response in Chronic Inflammatory Demyelinating Polyradiculoneuropathy-a Prospective Follow-Up", NEUROTHERAPEUTICS, ELSEVIER INC, US, vol. 15, no. 2, 12 February 2018 (2018-02-12), pages 439 - 451, XP036700629, ISSN: 1933-7213, [retrieved on 20180212], DOI: 10.1007/S13311-018-0609-4
- GODELAINE JORIS ET AL: "Abstract", BRAIN COMMUNICATIONS, vol. 3, no. 1, 16 January 2021 (2021-01-16), XP055848552, Retrieved from the Internet <URL:https://watermark.silverchair.com/fcab018.pdf?token=AQECAHi208BE49Ooan9kkhW_Ercy7Dm3ZL_9Cf3qfKAc485ysgAAAu0wggLpBgkqhkiG9w0BBwagggLaMIIC1gIBADCCAs8GCSqGSIb3DQEHATAeBglghkgBZQMEAS4wEQQMnRzzZvY-pw9LYca8AgEQgIICoFSh_nEOAR4qZJdd3sCZ_7FcIPxCJome3WYi-VN2Jt4WLUqZ3i2z1X5IpxpivgF3OgHtSPYPv0WMEtaZC5OmkeHjJO7R> DOI: 10.1093/braincomms/fcab018

## Description

### Background of the invention

"Chronic Inflammatory Polyradiculoneuropathy"" also called "Chronic Inflammatory Demyelinating Polyneuropathy" (CIDP) demonstrates considerable variation in clinical phenotype, disease progression and therapy response among patients [Mathey et al. (2015) J Neurol Neurosurg Psychiatry 86, 973-985; Viala et al. (2010) Peripher Nerv Syst 15, 50-56; Kuwabara et al. (2015) J Neurol Neurosurg Psychiatry 86, 1054-1059; Saperstein et al. (2001) Muscle Nerve 24, 311-324]. While some patients remain stable for years with no or only minimal immunomodulatory therapy, others progress more rapidly or experience lasting disability despite intensive long-term treatment [Mathey et al. (2015) J Neurol Neurosurg Psychiatry 86, 973-985; Berger et al. (2013) J Peripher Nerv Syst 18, 275-296; Hughes et al. (2008) Lancet Neurol 7, 136-144]. However, it is not yet possible to accurately predict how individual patients will progress. Also, it is estimated that eventually up to 30% of patients will not respond well to one of CIDP's main therapies (intravenous immunoglobulins, plasma exchange or steroids), necessitating therapy switch to halt or limit disease progression [Mathey et al. (2015) J Neurol Neurosurg Psychiatry 86, 973-985; Berger et al. (2013) J Peripher Nerv Syst 18, 275-296; Hughes et al. (2008) Lancet Neurol 7, 136-144]. Recently, paranodal autoantibodies associated with poor response to intravenous immunoglobulins and more aggressive disease course have been described in small subsets of CIDP patients [Cortese et al. (2020) Neurol Neuroimmunol Neuroinflamm 7, e639; Querol et al. (2015) Curr Opin Neurol 28, 474-479; Allen et al. (2020) JAMA Neurol. 77, 1159-1166]. To this date, however, reliable prognostic serum-based biomarkers applicable to the general population of CIDP patients are not yet available [Khoo et al. (2019) Biomark Res 7, 3].

In various neurological diseases, blood-based biomarkers that reflect disease progression have recently been gaining attention, including serum neurofilament light chain (sNfL) [Mariotto et al. (2018) J Peripher Nerv Syst 23, 174-177; van Lieverloo et al. (2019) J Peripher Nerv Syst 24, 187-194; Bridel et al. (2019) JAMA Neurol. 76(9), 1035-1048; Benatar et al. (2018) Ann Neurol 84, 130-139; Weydt et al. (2016) Ann Neurol 79, 152-158; Disanto et al. (2017) Ann Neurol 81, 857-870; Dalla Costa et al. (2019) Ann Neurol 85, 606-610; Loonstra et al. (2019) Ann Neurol 86, 322-324]. Neurofilaments are released into the blood and cerebrospinal fluid of patients with neurodegenerative diseases in whom they have been shown to be associated with various clinical features [Benatar et al. (2018) Ann Neurol 84, 130-139; Weydt et al. (2016) Ann Neurol 79, 152-158; Disanto et al. (2017) Ann Neurol 81, 857-870; Dalla Costa et al. (2019) Ann Neurol 85, 606-61; Loonstra et al. (2019) Ann Neurol 86, 322-324; Dalmau (2019) Neurol Neuroimmunol Neuroinflamm. 6, e601; De Schaepdryver et al. (2019) Ann Clin Transl Neurol 6, 1971-1979; Varhaug et al. (2019) Front Neurol 10, 338; Gille et al. (2019) Neuropathol Appl Neurobiol 45, 291-304 ; Poesen et al. (2017) Neurology 88, 2302-2309; Manouchehrinia et al. (2020) Neurology 294, e2457-e2467]. Moreover, recent studies showed significant associations between NfL and disease progression or therapy response in multiple sclerosis, a neuroinflammatory disease of the central nervous system [Disanto et al. (2017) Ann Neurol 81, 857-870 ; Dalla Costa et al. (2019) Ann Neurol 85, 606-610; Loonstra et al. (2019) Ann Neurol 86, 322-324; Manouchehrinia et al.(2020) Neurology 94, e2457-e2467; Canto et al. (2019) JAMA Neurol. 76, 1359-1366; Kuhle et al. (2016) Clin. chem. lab. medicine 54, 1655-1661; Oldoni et al. (2020) Ann Neurol. 8, 633-645].

There is a need for a prognostic biomarker of disease progression and/or therapy response in the neuroinflammatory peripheral nerve disorder CIDP.

### Summary of the invention

To investigate the prognostic value of serum neurofilament light chain (sNfL) in chronic inflammatory demyelinating polyneuropathy (CIDP) with respect to 1-year disease progression on Medical Research Council sum-score and therapy response over time.

In this retrospective study, sNfL was measured in clinical residual serum samples of 76 patients diagnosed with probable or definite CIDP according to EFNS/PNS diagnostic criteria. Logistic regression models were applied to assess sNfL as independent predictor for 1-year disease progression and therapy response over time.

In both univariate and multivariable (including demographics) models, elevated sNfL harbored increased odds for 1-year disease progression (respectively OR, 1.049; 95%CI, 1.022 - 1.084 and OR, 1.097; 95%CI, 1.045 - 1.169; both p =.001). Patients with sNfL levels above median cohort sNfL (28.3 pg/mL) had largely increased odds of 1-year disease progression (univariate: OR, 5.597; 95%CI, 1.590 - 26.457; p =.01; multivariable: OR, 6.572; 95%CI, 1.495 - 39.702; p =.02) and of requiring therapy switch due to insufficient treatment response (univariate: OR, 4.800; 95%CI, 1.622 - 16.442; p =.007; multivariable: OR, 6.441; 95%CI, 1.749 - 29.357; p =.009). Patients with sNfL levels below median cohort sNfL demonstrated increased odds of remaining stable while responding to therapy (univariate: OR, 6.337; 95%CI, 2.276 - 19.469; p <.001; multivariable: OR, 10.138; 95%CI, 2.801 - 46.404; p =.001).

sNfL was associated with both 1-year disease progression and therapy response over time in CIDP. Prospective studies utilizing well-characterized cohorts are warranted to confirm the added value of in CIDP's clinical practice.

The invention relates to methods for predicting disease progression or therapy response in a Chronic Inflammatory Demyelinating Polyneuropathy (CIDP) patient, comprising the steps of:
- determining in a sample of a CIDP patient the concentration of serum neurofilament light chain (sNfL),
- comparing the determined value with a reference value
- wherein an increased value compared to a reference value of individuals without disease progression is indicative for disease progression or is indicative for a need to switch therapy, and/or
- wherein a decreased value compared to a reference value of individuals without disease progression is indicative for responding to a therapy.
   or
- comparing the determined value with a median value of a group of patients comprising both patients likely to develop disease progression and patients likely not to develop disease progression
- wherein an value of sNfL above the median value of the group is indicative for disease progression or is indicative for a need to switch therapy, and/or
- wherein a value of sNfL below the median value is indicative for responding to a therapy.

In a typical embodiment, patients having sNfL levels above a median cohort sNfL of 28.3 pg/mL serum are being stratified as having 'high' sNfL, high sNfL.

Typically in these methods, wherein the CIDP patient is a patient undergoing therapy. Therapies include intravenous immunoglobulins, plasma exchange, a combination of intravenous immunoglobulins and steroids, and intravenous immunoglobulins and azathioprine.

"Undergoing therapy" can refer to receiving one of the above for a period of at least 2, 3, 4, 5 year, up to 6, 7, 8 or 9 year, for example between 4 and 5 years between 3.5 and 5.5 year, or between 3 and 6 year.

The terms "Chronic Inflammatory Polyradiculoneuropathy" and "Chronic Inflammatory Demyelinating Polyneuropathy" both abbreviated as CIDP, are both used for the same conditions.

The reference group can a group of CIDP patients undergoing therapy and comprising both individuals who will develop disease progression and individuals who will not developed disease progression. The outcome of disease progression in this reference may or may not be known at the moment of diagnosis. When such a refence group is used, elevated level of sNfL means a value above average, or above median value of the reference group.

In an alternative embodiment the reference group is based on samples collected over time and where disease progression has been documented.

Thus the comparison can be done using a reference of CIDP patients which did not develop disease progression over time (increase of sNfL is indicative of disease progression), or with a reference group which of CIDP patients which did not develop disease progression over time (similar value is indicative of disease progression).

sNfL is determined in a blood sample of the patient, typically in a serum sample. sNfL is determined and quantified by an antibody assay, such as electrochemilluminescence.

A reference value can be an average of sNfL in a cohort of CIDP patients prior to therapy.

The present invention relates to the use of sNfL in predicting disease progression and therapy response in CIDP.

The diagnosis pf patients predicted to have disease progression is used as a decision step to alter the therapy.

Patients predicted to have no disease progression can stay at the same therapy. "Disease progression" refers to a difference in clinical parameter over a time period of one year, typically a decrease of at least 4 points on an 80 point MRC sum score scale.

Whereas the prior art is not able to predict responders and non-responders to a therapy at the onset of the disease, the present inventions allows to make such decision after a certain time of therapy to allow to decide whether the same therapy can be used or whether a switch of therapy is to be considered.

### Figure legends

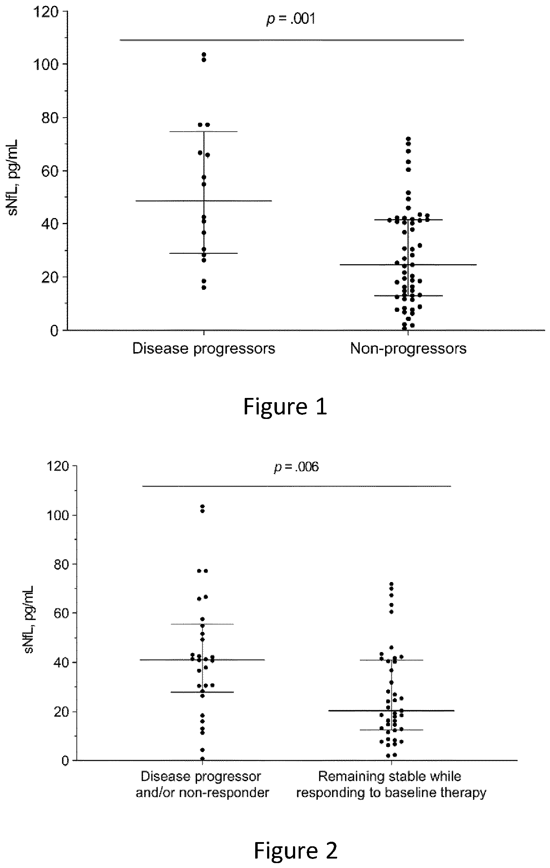

**Figure 1****.** Comparison of sNfL levels between 1-year disease progressors and non-progressors
   sNfL levels were significantly higher in disease progressors (median sNfL, 48.7 pg/mL; IQR, 45.8 pg/mL) compared to non-progressors (median sNfL, 26.4 pg/mL; IQR, 28.5 pg/mL), p = .001. Disease progression was defined as a decrease of at least four points on an 80-point MRC sum-score scale one year after sampling. Median and interquartile range are presented on top of the plot.
**Figure 2****.** Comparison of sNfL levels between 1-year disease progressors and/or therapy non-responders and other patients
   sNfL levels were significantly higher in the disease progressors and/or non-responders group (median sNfL, 41.0 pg/mL; IQR, 27.7 pg/mL) than in the group of patients who remained stable on MRC sum-score while responding to baseline therapy (median sNfL, 20.3 pg/mL; IQR, 28.3 pg/mL), p = .006. Median and interquartile range are presented on top of the plot.

### Detailed description

In recent years, the call for biomarkers to better predict disease progression or therapy response in CIDP has been increasing [Katzberg et al. (2017) Neurodegener Dis Manag 7, 147-156; Dalakas (2011) J Peripher Nerv Syst 16S1, 63-67; Lehmann (2019) J Neurol Neurosurg Psychiatry 90, 981-987; Oaklander et al. (2017) Cochrane Database Syst Rev 1, CD010369]. While progress has been made with the discovery of paranodal autoantibodies associated with more severe disease course and poor response to intravenous immunoglobulins, these markers have only been described in a small subset (<15%) of patients [Cortese et al. (2020) Neurol Neuroimmunol Neuroinflamm 7, e639; Querol et al. (2015) Curr Opin Neurol 28, 474-479; Allen et al. (2020) JAMA Neurol. 77, 1159-1166]. For the larger general CIDP population, prognostic serum-based biomarkers still present an unmet need. Recently, studies reported elevated sNfL levels in patients with peripheral neuropathies and some of these studies demonstrated significant cross-sectional associations between sNfL and disability (including in CIDP), hinting at the idea of sNfL as individual prognostic marker in these disorders [Mariotto et al. (2018) J Peripher Nerv Syst 23, 174-177; van Lieverloo et al. (2019) J Peripher Nerv Syst 24, 187-194 ; Axelsson et al. (2018) Acta Neurol Scand 138, 143-150 ; Bischof et al. (2018) Ann Rheum Dis 77, 1093-1094; Gaiottino et al. (2013) PLoS One 8, e75091; Sandelius et al. (2018) Neurology 90, e518-e524]. In current study, we investigated sNfL as predictor for 1-year disease progression and therapy response over time in 76 patients with CIDP.

In the present cohort, median sNfL levels were 28.3 pg/mL, similar to a previously reported median of 27.2 pg/mL in 24 patients with CIDP receiving immunomodulatory therapy at time of sampling [van Lieverloo et al. (2019) J Peripher Nerv Syst 24, 187-194]. We demonstrated that sNfL levels were associated with 1-year disease progression, which was in contrast to Van Lieverloo *et al.* who did not observe associations between sNfL and change in disability in patients in whom therapy was initiated at start of study [van Lieverloo et al. (2019) J Peripher Nerv Syst 24, 187-194]. This discrepancy is likely caused through differences in study design: while we defined disease progression as a decrease of at least four points on an 80-point MRC sum-score scale over a 1-year period, Van Lieverloo *et al.* measured disability by various measurements including a 60-point MRC sum-score scale over a shorter period of six months [van Lieverloo et al. (2019) J Peripher Nerv Syst 24, 187-194]. As the 'standard' 60-point scale has been shown to be less sensitive to changes in patients with only mild or distal CIDP [Katzberg et al. (2017) Neurodegener Dis Manag 7, 147-156], this indicates that the use of the 80-point scale - which assesses two additional distal muscle groups - enabled to detect smaller changes in disability. Another possible explanation might be situated in inclusion criteria: while in the study of Van Lieverloo *et al.* most patients in whom no association between sNfL and change in disability was found were newly diagnosed and treatment naive [van Lieverloo et al. (2019) J Peripher Nerv Syst 24, 187-194], the majority of the patients (61/76) were already receiving therapy at time of sampling. Since so far predominantly the short-term efficacy of CIDP therapies has been established [Oaklander et al. (2017) Cochrane Database Syst Rev 1, CD010369 ; Eftimov et al. (2013) Cochrane Database Syst Rev (12) CD001797], with only a limited number of studies addressing long-term efficacy, it may be possible that treatment naive patients still experience larger treatment effects and thus less short-term disease progression than patients who have been receiving treatment for a longer period of time as previously has been described for multifocal motor neuropathy, another neuroinflammatory peripheral nerve disease [Van den Berg et al. (1998) Brain 121, 421-428].

Van Lieverloo *et al.* (cited above) previously reported an association between CIDP patients not responding to therapy and increased sNfL levels at time of therapy assessment but, in contrast to current study, the authors did not investigate the predictive value of sNfL for therapy response. The present results now demonstrate that patients with sNfL levels above the median cohort sNfL had increased odds of requiring therapy switch due to insufficient treatment response. As such, sNfL may hold value in the clinical decision making of CIDP by identifying those patients who would eventually require treatment switch or who should immediately be started on more intensive treatment (e.g. combination therapy). The present invention shows that sNfL was able to prognostically differentiate patients remaining stable on MRC sum-score while responding to baseline therapy from disease progressors and/or non-responders (i.e. a combined approach) with sNfL levels below the median cohort sNfL being associated with greatly increased odds of remaining stable in this analysis. As such, the results of this combined approach further substantiate the association of sNfL with 1-year disease progression and therapy response over time.

Due to retrospective study design, the included cohort of CIDP patients was rather heterogeneous with respect to demographic variables. While mostly unassociated with sNfL levels, this heterogeneity was countered by including these demographics in the multivariable models being used. The retrospective design also implied that only those patients could be included for whom a residual serum sample was available, thus limiting the cohort size. Lastly, during sNfL measurements, 18 samples were encountered with concentrations below the previously reported LLOQ of the electrochemiluminescence assay (15.6 pg/mL) [Kuhle et al. (2016) Clin Chem Lab Med 54, 1655-1661].

In summary, the present retrospective longitudinal study showed that sNfL was associated with both 1-year disease progression on MRC sum-score and therapy response over time in CIDP.

### Examples

### Methods

### Patient inclusion

For this single-center retrospective longitudinal study, adult patients had to comply with following inclusion criteria: 1) having received a probable or definite CIDP diagnosis according to the European Federation of Neurological Societies/Peripheral Nerve Society diagnostic criteria by a trained neurologist; 2) having a clinical residual serum sample stored at the biobank of the University Hospitals Leuven from 2009 the earliest; 3) having been followed-up at the University Hospitals Leuven NeuroMuscular Reference Center (NMRC) for at least one year after blood sampling and 4) availability of demographic and clinical data in electronic medical records, including information on therapy response and/or disease progression. Patients who experienced around time of sampling any event that might influence sNfL levels (e.g. stroke, infection, documented head trauma, etc.) were not considered.

### Patient classification

Included patients were classified according to both 1-year disease progression ('progressor' or 'non-progressor') and therapy response over time ('responder' or 'non-responder') via retrospective study of medical records:
1-year disease progression was defined as a decrease of at least four points (the minimal clinically important difference [Merkies et al. (2010) J Neurol Neurosurg Psychiatry 81, 1194-1199; Merkies et al. (2017) J Peripher Nerv Syst 22, 149-152]) on an 80-point Medical Research Council (MRC) sum-score scale one year after serum sampling (assessed by consulting the medical record closest to one year after sampling) compared to the MRC sum-score measured at time of sampling ('baseline' MRC sum-score). The 80-point MRC sum-score, previously applied in clinical trials [van Schaik et al. (2018) Lancet Neurol 17, 35-46; Leger et al. (2013) J Peripher Nerv Syst 18, 130-140], is the sum of scores for eight bilateral (left and right side) muscle groups each rated between zero (no visible contraction) to five (normal movement) and is used at the University Hospitals Leuven NMRC to follow-up patients. Higher scores indicate greater muscle contraction/limb movement.

Therapy response over time was assessed for each patient as followed: we examined what treatment (intravenous immunoglobulins, plasma exchange, steroids, immunosuppressive drugs or a combination of therapies) was utilized at time of sampling ('baseline' treatment). When no therapy was yet initiated, the treatment established closest to time of sampling was designated baseline treatment. When, by clinical judgement of sufficient therapy response, a therapy switch from this baseline treatment was not deemed necessary during follow-up (assessed by consulting the most recent medical record available for each patient with minimum interval of one year between therapy initiation and response assessment), patients were classified as 'therapy responders'. However, when a therapy switch was required due to insufficient treatment effect, patients were classified as 'non-responders'.

### sNfL measurements

Peripheral blood sampling and isolation of serum was performed according to standardized operating procedures during routine clinical practice in diagnostic work-up and follow-up of patients. As part of routine practice, residual material was stored in the biobank of the University Hospitals Leuven clinical laboratory at -20°C. For each patient that satisfied inclusion criteria, the earliest sample available in the biobank (i.e. closest to diagnosis) was sorted out for sNfL measurement. sNfL was measured using a previously established electrochemiluminescence assay [Limberg et al. (2016) J. Methods Mol Biol 1304, 93-98]. The signal was measured using a Meso Quickplex SQ120 multiplex imager (Mesoscale Discovery, Rockville, MD). Samples were measured in duplicate and all coefficients of variation of duplicate determinations were less than 10%. Results below the previously reported lower limit of quantification (LLOQ) of the assay (15.6 pg/mL) [Kuhle et al. (2016) Clin Chem Lab Med 54, 1655-1661] were not discarded or transformed to a variation of the LLOQ (e.g. LLOQ, LLOQ/√2 etc.) but were used as such in order to improve performance of statistical models [Keizer et al. (2015) Pharmacol Res Perspect 3, e00131].

### Statistical analysis

Univariate and multivariable linear least square regression models were used to investigate associations between sNfL levels and demographics (age, disease duration, CIDP phenotype, sex) and MRC sum-score at time of sampling. Regression residuals showed no heteroscedasticity or important deviations from normality. sNfL levels were compared between 1-year disease progression groups (progressors vs. non-progressors) and therapy response groups (responders vs. non-responders) using either student's t tests or non-parametric Mann-Whitney U tests based on distribution of data as assessed by Shapiro-Wilk test.

Logistic regression models were used to evaluate sNfL as predictor of 1-year disease progression and of therapy response over time. Both univariate and multivariable logistic regression models were used, with multivariable models including the demographics age, disease duration, sex and CIDP phenotype (classical or atypical) to correct for demographic heterogeneity in the cohort. Patients with missing data regarding 1-year disease progression or therapy response over time were excluded for the associated statistical analysis. Logistic regression models were also used to evaluate the ability of sNfL to differentiate those patients who remained stable on MRC sum-score while responding to therapy (i.e. responding non-progressors) from others (i.e. progressors and/or non-responders). In all models, sNfL was evaluated both as continuous variable and as binary variable through stratification of patients as having low or high sNfL based on the median cohort sNfL. Receiver operating characteristic (ROC) curve analyses were used to visualize models' performances. Statistical analyses were performed using IBM SPSS (V26.0, IBM Corp., Armonk, NY, USA). α = .05 was used as cutoff for significance. As this was an exploratory study, no corrections for multiple comparisons were made [Althouse (2016) Ann Thorac Surg 101, 1644-1645; Bender & Lange (2001) J Clin Epidemiol 54, 343-349; Cao & Zhang (2014) JAMA 312, 543-544]. A power calculation could not be performed due to insufficient data on sNfL in CIDP. However, the present sample size is similar to those generally used in the field of biomarker research in CIDP. All tests were 2-sided and confidence intervals were reported as profile likelihood intervals [Royston (2018) Stata J. 7, 376-387].

### Standard protocol approvals, registrations and patient consents

All patients agreed with storage of clinical residual material for future research by opting-out agreement. Ethical approval for the study was granted by Ethical Committee Research UZ/KU Leuven (S62265).

### Results

### Demographic and clinical characteristics of the CIDP cohort

76 patients with CIDP were included with a mean (SD) age of 61.5 (11.7) years. All patients were of non-Hispanic Caucasian descent and 11/76 (14.5%) were female. Cohort demographics and clinical features are summarized in Table 1. Information regarding 1-year disease progression was available for 71/76 patients (93.4%) while information regarding therapy response was available for all patients.

**Table 1. Demographic and clinical characteristics of the CIDP cohort**

| **Variable** | | **N = 76** |
|---|---|---|
| Age at time of sampling, mean (SD), years | | 61.5 (11.7) |
| Female, No. (%) | | 11 (14.5) |
| Timespan between self-reported symptom onset and time of sampling, median (IQR), years | | 6.0 (6.9) |
| Time in follow-up at University Hospitals Leuven NMRC at last consult, median (IQR), years | | 6.9 (6.7) |
| Timespan between time of sampling and 1-year disease progression assessment, median (IQR), months | | 12.4 (1.7) |
| Timespan between time of sampling and therapy assessment at last consult, median (IQR), years | | 4.5 (4.2) |
| EFNS/PNS diagnostic criteria, No. (%) | | |
| | Definite | 63 (82.9) |
| | Probable | 13 (17.1) |
| Phenotype, No. (%) | | |
| | Classic CIDP | 58 (76.3) |
| | Atypical CIDP | 18 (23.7) |
| | Acute onset | 6 |
| | DADS | 3 |
| | MADSAM | 8 |
| | Pure sensory | 1 |
| 1-year disease progression, No. (%) | | |
| | Progressors | 16 (21) |
| | Non-progressors | 55 (72.4) |
| | Unknown (missing data) | 5 (6.6) |
| Therapy response over time, No. (%) | | |
| | Responders | 55 (72.4) |
| | Non-responders | 21 (27.6) |
| MRC sum-score at time of sampling, median (IQR)^{a} | | 77 (6) |
| MRC sum-score one year after sampling, median (IQR)^{b} | | 77 (8) |
| sNfL, median (IQR), pg/mL | | 28.3 (26.4) |

| | | |
|---|---|---|
| ^{a} Available for 73/76 patients; ^{b} Available for 71/76 patients. Abbreviations: DADS, distal acquired demyelinating symmetric neuropathy; EFNS/PNS, European Federation of Neurological Societies/ Peripheral Nerve Society; MADSAM, multifocal acquired demyelinating sensory and motor neuropathy; MRC, Medical Research Council; sNfL, serum neurofilament light chain; NMRC, NeuroMuscular Reference Center. | | |

### Associations between sNfL and demographic and clinical characteristics

Median cohort sNfL concentration was 28.3 pg/mL (Interquartile range (IQR), 26.4 pg/mL) with 18 patients having sNfL levels below the LLOQ (range, 1-15 pg/mL). sNfL was correlated with age at time of sampling (β, 1.183; 95% CI, 0.822 - 1.545; *p* < .001), but not with disease duration, sex, CIDP phenotype or baseline MRC sum-score (Table 2). A multivariable model including demographics and baseline MRC sum-score confirmed the correlation between sNfL and age (β, 1.251; 95% CI, 0.865 - 1.638; p < .001; Table 2).

### sNfL as predictor of 1-year disease progression

Patients were classified as 1-year disease progressor when compared to baseline MRC sum-score, a decrease of four or more points was seen one year after sampling. Data regarding disease progression was missing for 5/76 patients and these patients were excluded from the analysis. 16/71 (22.5%) patients were classified as disease progressors with a median (IQR) decrease in MRC sum-score of six (2.25) points and sNfL levels were increased in these patients compared to non-progressors (respectively median (IQR) sNfL of 48.7 (45.8) and 24.6 (28.5) pg/mL; p = .001) (Figure 1).

sNfL as continuous variable was found to be associated with 1-year disease progression, both in univariate (odds ratio (OR), 1.049; 95% CI, 1.022 - 1.084; p = .001) and multivariable logistic regression models (OR, 1.097; 95% CI, 1.045 - 1.169; *p* = .001) (Table 3). ROC curve analysis resulted in an area under the curve (AUC) of 0.765 (95% CI, 0.636 - 0.893; *p* < .001). When sNfL was included in the logistic regression models as binary variable, with patients having sNfL levels above the median cohort sNfL (28.3 pg/mL) being stratified as having 'high' sNfL, high sNfL levels were associated with strongly increased odds of 1-year disease progression (univariate: OR, 5.597; 95% CI, 1.590 - 26.457; *p* = .01; multivariable: OR, 6.572; 95% CI, 1.495 - 39.702; *p* = .02) (Table 3). Age and disease duration were also significantly associated with 1-year disease progression in the multivariable continuous NfL logistic regression model, but not in the multivariable binary NfL logistic regression model (Table 3).

*sNfL as predictor of requiring therapy switch due to insufficient treatment response* 61/76 (80.3%) patients were receiving therapy at time of sampling, while for 14/76 (18.4%) patients intravenous immunoglobulin therapy was initiated within one year after sampling. One patient did not receive any therapy throughout follow-up (>10 years) but remained stable without treatment. sNfL levels did not differ between patients receiving therapy at time of sampling versus those who did not (respectively median (IQR) sNfL 28.3 (25.6) and 26.3 (30.3) pg/mL; *p* = .98). In total, baseline treatment to assess therapy response was intravenous immunoglobulin therapy for 60/76 patients (79%), plasma exchange for 7/76 patients (9.2%), a combination of intravenous immunoglobulins and steroids for 3/76 patients (3.9%) and a combination of intravenous immunoglobulins and azathioprine for 5/76 patients (6.6%). Median (IQR) time span over which therapy response was assessed was 4.5 (4.2) years with a minimum of one year. 21/76 patients (27.6%) were classified as non-responders when, compared to baseline therapy, a treatment switch was required throughout follow-up as a result of insufficient treatment effect (Table 1). sNfL levels tended to be increased in non-responders but did not significantly differ from the levels observed in therapy responders (respectively median (IQR) sNfL of 41.2 (33.3) and 25.2 (22.9) pg/mL; *p* = .06).

**Table 2. Associations between sNfL levels and clinical and demographic characteristics**

| **Variable** | **sNfL, median (IQR), pg/mL** | **Univariate** | | | **Multivariable** | | |
|---|---|---|---|---|---|---|---|
| | | **β** | **95% CI** | ***p*** | **β** | **95% CI** | ***p*** |
| Age, years | - | 1.183 | 0.822 - 1.545 | < .001 | 1.251 | 0.865 - 1.638 | < .001 |
| Sex | | | | | | | |
| Female (11) | 24.6 (42.0) | - | - | - | - | - | - |
| Male (65) | 28.3 (26.5) | 3.665 | -11.256 - 18.586 | .63 | -5.571 | -17.733 - 6.592 | .36 |
| Disease duration, years | - | 0.717 | -0.199 - 1.634 | .12 | -0.156 | -0.943 - 0.632 | .70 |
| CIDP Phenotype | | | | | | | |
| Typical (58) | 26.3 (26.5) | - | - | - | - | - | - |
| Atypical (18) | 38.7 (46.3) | 6.195 | -6.092 - 18.482 | .32 | 6.263 | -3.768 - 16.294 | .22 |
| MRC sum-score at time of sampling (73^{a}) | - | -0.484 | -1.082 - 0.115 | .11 | -0.426 | -0.910 - 0.058 | .08 |

The number of patients for each category is indicated in parenthesis. ^{a} MRC-sum score at time of sampling was only available for 73/76 patients. Abbreviations: CIDP, Chronic Inflammatory Demyelinating Polyneuropathy; MRC, Medical Research Council; sNfL, serum neurofilament light chain

**Table 3. Logistic regression models for sNfL as predictor of 1-year disease progression**

| **Variable** | **Univariate** | | | **Multivariable (continuous sNfL)** | | | **Multivariable (binary sNfL)** | | |
|---|---|---|---|---|---|---|---|---|---|
| | **OR** | **95% CI** | ***p*** | **OR** | **95% CI** | ***p*** | **OR** | **95% CI** | ***p*** |
| Age, years | 1.019 | 0.972 - 1.072 | .44 | 0.902 | 0.816 - 0.981 | .03 | 0.976 | 0.914 - 1.036 | .43 |
| Disease duration, years | 1.110 | 1.011 - 1.232 | .03 | 1.172 | 1.039 - 1.358 | .02 | 1.101 | 0.997 - 1.234 | .07 |
| Phenotype, atypical | 1.629 | 0.444 - 5.480 | .44 | 0.607 | 0.108 - 2.854 | .55 | 1.075 | 0.243 - 4.185 | .92 |
| Sex, male | 0.738 | 0.182 - 3.724 | .68 | 0.739 | 0.138 - 4.439 | .73 | 0.629 | 0.131 - 3.496 | .57 |
| sNfL, pg/mL | 1.049 | 1.022 - 1.084 | .001 | 1.097 | 1.045 - 1.169 | .001 | - | - | - |
| High sNfL^{a} | 5.597 | 1.590 - 26.457 | .01 | - | - | - | 6.572 | 1.495 - 39.702 | .02 |

| | | | | | | | | | |
|---|---|---|---|---|---|---|---|---|---|
| ^{a} Based on median cohort sNfL value (28.3 pg/mL) patients were binarily categorized as having 'high' or 'low' sNfL values. Data regarding disease progression was missing for 5/76 patients and these patients were not included in the analysis. 16/71 (22.5%) patients showed a decrease of four or more points on an 80-point MRC sum-score scale one year after sampling and were classified as disease progressors. Abbreviations: OR, odds ratio; sNfL, serum neurofilament light chain. | | | | | | | | | |

sNfL as continuous variable was not significantly associated with requiring therapy switch due to insufficient treatment response (univariate model: OR, 1.021; 95% CI, 1.000 - 1.045; *p* = .06; multivariable: OR, 1.024; 95% CI, 0.997 - 1.054; *p* = .09) (Table 4). ROC curve analysis resulted in an AUC of 0.643 (95% CI, 0.497 - 0.790; *p* = .06). However, when sNfL was included as binary variable by stratifying patients according to the median cohort sNfL (28.3 pg/mL), high sNfL levels were significantly associated with increased odds of requiring therapy switch, both in univariate (OR, 4.800; 95% CI, 1.622 - 16.442; *p* = .007) and multivariable (OR, 6.441; 95% CI, 1.749 - 29.357; *p* = .009) models (Table 4).

*sNfL as predictor of remaining stable on MRC sum-score while responding to therapy* As sNfL was associated with both 1-year disease progression and therapy response, we next evaluated in a combined approach whether sNfL levels could also differentiate between patients who remained stable on MRC sum-score while responding to baseline therapy (i.e. responding non-progressors) versus others (i.e. patients showing 1-year disease progression and/or insufficient response to baseline therapy). 5/76 patients were excluded from analysis due to missing data regarding disease progression. 41/71 patients (57.7%) remained stable on MRC sum-score while responding to baseline therapy, while 30/71 patients (42.3%) were disease progressor (11 patients), therapy non-responder (14 patients) or both (five patients). sNfL levels were higher in the disease progressors and/or non-responders group than in patients who remained stable while responding to therapy (respectively median (IQR) sNfL of 41.0 (27.7) and 20.3 (28.3) pg/mL); *p* = .006; Figure 2).

Increasing sNfL levels decreased the odds of remaining stable on MRC sum-score while responding to baseline therapy (univariate: OR, 0.968; 95% CI, 0.943 - 0.990; *p* = .008; multivariable: OR, 0.951; 95% CI, 0.915 - 0.983; *p* = .005) (Table 5). ROC curve analysis resulted in an AUC of 0.692 (95% CI, 0.565 - 0.819; *p* = .003). When sNfL was included in the logistic regression models as binary variable, with patients having sNfL levels below the median cohort sNfL (28.3 pg/mL) being stratified as having 'low' sNfL, low sNfL corresponded with greatly increased odds of remaining stable while responding to therapy (univariate: OR, 6.337; 95% CI, 2.276 - 19.469; *p* < .001; multivariable: OR, 10.138; 95% CI, 2.801 - 46.404; *p* = .001) (Table 5).

**Table 4. Logistic regression models for sNfL as predictor of requiring therapy switch due to insufficient treatment response**

| **Variable** | **Univariate** | | | **Multivariable (continuous sNfL)** | | | **Multivariable (binary sNfL)** | | |
|---|---|---|---|---|---|---|---|---|---|
| | **OR** | **95% CI** | ***p*** | **OR** | **95% CI** | ***p*** | **OR** | **95% CI** | ***p*** |
| Age, years | 1.018 | 0.975 - 1.065 | .42 | 0.987 | 0.928 - 1.046 | .66 | 0.979 | 0.918 - 1.036 | .48 |
| Disease duration, years | 0.999 | 0.908 - 1.090 | .98 | 0.986 | 0.890 - 1.084 | .78 | 0.973 | 0.875 - 1.070 | .59 |
| Phenotype, atypical | 2.000 | 0.632 - 6.128 | .23 | 1.926 | 0.567 - 6.372 | .28 | 1.753 | 0.475 - 6.197 | .39 |
| Sex, male | 4.444 | 0.771 - 84.321 | .17 | 4.871 | 0.792 - 94.954 | .15 | 5.212 | 0.828 - 102.20 | .14 |
| sNfL, pg/mL | 1.021 | 1.000 - 1.045 | .06 | 1.024 | 0.997 - 1.054 | .09 | - | - | - |
| High sNfL^{a} | 4.800 | 1.622 - 16.442 | .007 | - | - | - | 6.441 | 1.749 - 29.357 | .009 |

| | | | | | | | | | |
|---|---|---|---|---|---|---|---|---|---|
| ^{a} Based on median cohort sNfL (28.3 pg/mL) patients were binarily categorized as having 'high' or 'low' sNfL values. 21/76 (27.6%) patients required therapy switch during follow-up due to insufficient treatment response. Abbreviations: OR, odds ratio; sNfL, serum neurofilament light chain. | | | | | | | | | |

**Table 5. Logistic regression models for sNfL as predictor of remaining stable on MRC sum-score while responding to baseline therapy**

| **Variable** | **Univariate** | | | **Multivariable (continuous sNfL)** | | | **Multivariable (binary sNfL)** | | |
|---|---|---|---|---|---|---|---|---|---|
| | **OR** | **95% CI** | ***p*** | **OR** | **95% CI** | ***p*** | **OR** | **95% CI** | ***p*** |
| Age, years | 0.982 | 0.941 - 1.022 | .38 | 1.055 | 0.993 - 1.131 | .10 | 1.047 | 0.988 - 1.123 | .15 |
| Disease duration, years | 0.934 | 0.852 - 1.015 | .12 | 0.920 | 0.819 - 1.015 | .12 | 0.934 | 0.835 - 1.030 | .20 |
| Phenotype, atypical | 0.773 | 0.256 - 2.355 | .65 | 1.243 | 0.352 - 4.783 | .74 | 1.122 | 0.305 - 4.363 | .86 |
| Sex, male | 0.458 | 0.093 - 1.759 | .28 | 0.345 | 0.063 - 1.500 | .18 | 0.380 | 0.069 - 1.682 | .22 |
| sNfL, pg/mL | 0.968 | 0.943 - 0.990 | .008 | 0.951 | 0.915 - 0.983 | .005 | - | - | - |
| Low sNfL^{a} | 6.337 | 2.276 - 19.469 | <.001 | - | - | - | 10.138 | 2.801 - 46.404 | .001 |

| | | | | | | | | | |
|---|---|---|---|---|---|---|---|---|---|
| ^{a} Based on median cohort sNfL value (28.3 pg/mL) patients were binarily categorized as having 'high' or 'low' sNfL values. Data regarding disease progression was missing for 5/76 patients and these patients were not included in the analysis. 41/71 patients (57.7%) remained stable on MRC sum-score while responding to baseline therapy. Abbreviations: OR, odds ratio; sNfL, serum neurofilament light chain. | | | | | | | | | |

## Claims

1. A method for predicting disease progression or therapy response in a Chronic Inflammatory Demyelinating Polyneuropathy (CIDP) patient, comprising the steps of:
- determining in a sample of a CIDP patient the concentration of serum neurofilament light chain (sNfL),
- comparing the determined value with a reference value,
wherein an increased value compared to a reference value of individuals without disease progression is indicative for disease progression or is indicative for a need to switch therapy, and/or
- wherein a decreased value compared to a reference value of individuals without disease progression is indicative for responding to a therapy,
or
- comparing the determined value with a median value of a group of patients comprising both patients likely to develop disease progression and patients likely not to develop disease progression,
- wherein an value of sNfL above the median value of the group is indicative for disease progression or is indicative for a need to switch therapy, and/or
- wherein a value of sNfL below the median value is indicative for responding to a therapy.

2. The method according to claim 1, wherein the sample is serum.

3. The method according to claim 1 or 2, wherein sNfL is determined via an immunoassay.

4. The method according to any one of claims 1 to 3, wherein the CIDP patient is a patient undergoing therapy.

5. The method according to claim 4, wherein the therapy is selected from the group consisting of the administration of intravenous immunoglobulins, plasma exchange, the administration of a combination of intravenous immunoglobulins and steroids, and the intravenous administration of immunoglobulins and azathioprine.

6. The method according to any one of claims 1 to 5, wherein the therapy has been performed for at least 2 years.

7. The method according to any one of claims 1 to 6, wherein disease progression is determined as a difference in a clinical parameter over a time period of one year.

8. The method according to any one of claims 1 to 7, wherein disease progression is a decrease of at least 4 points on an 80 point Medical Research Council (MRC) sum score scale.

9. Use of serum neurofilament light chain (sNfL) as a marker for predicting disease progression or therapy response in a Chronic Inflammatory Demyelinating Polyneuropathy (CIDP) patient.

## Patentansprüche

1. Verfahren zum Vorhersagen von Krankheitsprogression oder Therapieansprechen bei einem Patienten mit chronisch inflammatorischer demyelinisierender Polyneuropathie (CIDP), umfassend die Schritte:
- Bestimmen, in einer Probe eines CIDP-Patienten, der Konzentration einer Serum-Neurofilament-Leichtkette (sNfL),
- Vergleichen des bestimmten Wertes mit einem Referenzwert,
wobei ein erhöhter Wert im Vergleich zu einem Referenzwert von Individuen ohne Krankheitsprogression auf Krankheitsprogression hinweist oder auf eine Notwendigkeit hinweist, die Therapie zu wechseln, und/oder
- wobei ein verringerter Wert im Vergleich zu einem Referenzwert von Individuen ohne Krankheitsprogression zum Ansprechen auf eine Therapie hinweist,
oder
- Vergleichen des bestimmten Wertes mit einem Medianwert einer Gruppe von Patienten, umfassend sowohl Patienten, bei denen wahrscheinlich ist, dass sie Krankheitsprogression entwickeln, als auch Patienten, bei denen wahrscheinlich ist, dass sie Krankheitsprogression nicht entwickeln,
- wobei ein Wert von sNfL über dem Medianwert der Gruppe auf Krankheitsprogression hinweist oder auf eine Notwendigkeit hinweist, die Therapie zu wechseln, und/oder
- wobei ein Wert von sNfL unter dem Medianwert zum Ansprechen auf eine Therapie hinweist.

2. Verfahren nach Anspruch 1, wobei die Probe Serum ist.

3. Verfahren nach Anspruch 1 oder 2, wobei sNfL über einen Immunassay bestimmt wird.

4. Verfahren nach einem der Ansprüche 1 bis 3, wobei der CIDP-Patient ein Patient ist, der sich einer Therapie unterzieht.

5. Verfahren nach Anspruch 4, wobei die Therapie aus der Gruppe ausgewählt ist, bestehend aus der Verabreichung von intravenösen Immunglobulinen, einem Plasmaaustausch, der Verabreichung einer Kombination aus intravenösen Immunglobulinen und Steroiden und der intravenösen Verabreichung von Immunglobulinen und Azathioprin.

6. Verfahren nach einem der Ansprüche 1 bis 5, wobei die Therapie seit mindestens 2 Jahren durchgeführt wird.

7. Verfahren nach einem der Ansprüche 1 bis 6, wobei Krankheitsprogression als ein Unterschied bei einem klinischen Parameter über einen Zeitraum von einem Jahr bestimmt wird.

8. Verfahren nach einem der Ansprüche 1 bis 7, wobei Krankheitsprogression eine Verringerung von mindestens 4 Punkten auf einer 80-Punkte-Summenscore-Skala des Medical Research Council (MRC) ist.

9. Verwendung der Serum-Neurofilament-Leichtkette (sNfL) als ein Marker zum Vorhersagen von Krankheitsprogression oder Therapieansprechen bei einem Patienten mit chronisch inflammatorischer demyelinisierender Polyneuropathie (CIDP).

## Revendications

1. Procédé de prédiction d'une progression de la maladie ou d'une réponse au traitement chez un patient atteint de polyneuropathie inflammatoire démyélinisante chronique (PIDC), comprenant les étapes consistant à :
- déterminer dans un échantillon d'un patient atteint de PIDC la concentration de la chaîne légère de neurofilament sérique (sNfL),
- comparer la valeur déterminée à une valeur de référence,
dans lequel une valeur augmentée par rapport à une valeur de référence d'individus sans progression de la maladie indique une progression de la maladie ou indique une nécessité de changer de traitement, et/ou
- dans lequel une valeur diminuée par rapport à une valeur de référence d'individus sans progression de la maladie indique une réponse à un traitement,
ou
- comparer la valeur déterminée à une valeur médiane d'un groupe de patients comprenant à la fois des patients susceptibles de développer une progression de la maladie et des patients susceptibles de ne pas développer de progression de la maladie,
- dans lequel une valeur de sNfL supérieure à la valeur médiane du groupe indique une progression de la maladie ou indique la nécessité de changer de traitement, et/ou
- dans lequel une valeur de sNfL inférieure à la valeur médiane indique une réponse à un traitement.

2. Procédé selon la revendication 1, dans lequel l'échantillon est du sérum.

3. Procédé selon la revendication 1 ou 2, dans lequel la sNfL est déterminée par l'intermédiaire d'un immunoessai.

4. Procédé selon l'une quelconque des revendications 1 à 3, dans lequel le patient atteint de PlOC est un patient en cours de traitement.

5. Procédé selon la revendication 4, dans lequel le traitement est choisi dans le groupe constitué de l'administration d'immunoglobulines intraveineuses, d'échange de plasma, de l'administration d'une combinaison d'immunoglobulines intraveineuses et de stéroïdes, et de l'administration intraveineuse d'immunoglobulines et d'azathioprine.

6. Procédé selon l'une quelconque des revendications 1 à 5, dans lequel le traitement a été réalisé pendant au moins 2 ans.

7. Procédé selon l'une quelconque des revendications 1 à 6, dans lequel la progression de la maladie est déterminée comme une différence dans un paramètre clinique sur une période de temps d'un an.

8. Procédé selon l'une quelconque des revendications 1 à 7, dans lequel la progression de la maladie est une diminution d'au moins 4 points sur une échelle de score total du conseil de la recherche médicale (MRC) de 80 points.

9. Utilisation d'une chaîne légère de neurofilament sérique (sNfL) comme un marqueur permettant de prédire une progression de la maladie ou une réponse au traitement chez un patient atteint de polyneuropathie inflammatoire démyélinisante chronique (PIDC).
